Europäisches Patentamt

European Patent Office

Office européen des brevets

(19)

(11) Publication number : **0 404 334 B1**

(12) **EUROPEAN PATENT SPECIFICATION**

(45) Date of publication of patent specification :
02.02.94 Bulletin 94/05

(51) Int. Cl.⁵ : **A61K 9/12**

(21) Application number : **90305248.8**

(22) Date of filing : **15.05.90**

(54) **Aerosol compositions for topical application.**

(30) Priority : **19.05.89 GB 8911529**
**13.11.89 GB 8925605**

(43) Date of publication of application :
**27.12.90 Bulletin 90/52**

(45) Publication of the grant of the patent :
**02.02.94 Bulletin 94/05**

(84) Designated Contracting States :
**AT BE CH DE DK ES FR GB GR IT LI LU NL SE**

(56) References cited :
CH-A- 413 488
CH-A- 634 480
GB-A- 1 099 722
US-A- 4 134 968
US-A- 4 450 151
US-A- 4 716 032
O.-A. NEUMÜLLER "Römpps Chemie-
Lexikon", 8th edition, vol. 4, 1985 FRANCK-
H'SCHE VERLAGSHANDLUNG STUTTGART
page 2579
PATENT ABSTRACTS OF JAPAN, unexamined
applications, C field, vol. 11, no. 214, July 10,
1987 THE PATENT OFFICE JAPANESE GOV-
ERNMENT page 68 C 434

(56) References cited :
Seifen- le-Fette-Wachse, vol. 111, 1985 Verlag
fUr chemische In- dustrie H. ZIOLKOWSKY KG
AUGSBURG pages 179-180
PATENT ABSTRACTS OF JAPAN, unexamined
applications, C field, vol. 11, no. 206, July 3,
1987 THE PATENT OFFICE JAPANESE GOV-
ERNMENT page 42 C 433
PATENT ABSTRACTS OF JAPAN, unexamined
applications, C field, vol. 6, no. 11, January 22,
1982 THE PATENT OFFICE JAPANESE GOV-
ERNMENT page 66 C 88

(73) Proprietor : **Beecham Group p.l.c.**
**SB House Great West Road**
**Brentford Middlesex TW8 9BD (GB)**

(72) Inventor : **Brobyn, Susan Elizabeth SB**
**Consumer Brands**
**Res. and Techn. Services Centre St. George's**
**Ave.**
**Weybridge Surrey KT13 0DE (GB)**
Inventor : **Mackie, Robert Duncan SB**
**Consumer Brands**
**Res. and Techn. Services Centre St. George's**
**Ave.**
**Weybridge Surrey KT13 0DE (GB)**

(74) Representative : **Lockwood, Barbara Ann et al**
**SmithKline Beecham, Corporate Patents,**
**Great Burgh, Yew Tree Bottom Road**
**Epsom, Surrey KT18 5XQ (GB)**

## Description

The present invention relates to a composition suitable for use as a so-called aerosol "freeze spray".

So-called "freeze sprays" are aerosol compositions which, when sprayed onto a patient's skin, have a cooling or "freezing" effect on that part of the body. They are used in the alleviation of, for example, muscular pain, rheumatic pain, lumbago, sciatica, and neuritis. Such freeze sprays currently comprise a high proportion of, or in some cases consist entirely of, chlorofluorocarbons (CFC's).

There has recently been considerable concern about the use of chlorofluorocarbons, and their contribution to pollution of the environment and, in particular, their effect in causing depletion of the ozone layer in the upper atmosphere. There has therefore been considerable effort made to reduce the use of chlorofluorocarbons in, inter alia, aerosol compositions by the use of alternative propellants and other components.

Considerable advancement has been made toward reducing or eliminating the use of chlorofluorocarbons in many types of aerosol composition, but, despite considerable effort in that direction, it has not hitherto been possible to find alternative components suitable for use in freeze sprays. This is because the chlorofluorocarbons themselves are largely responsible for the freezing effect on the skin and also have the useful property of being good solvents. The alternative propellants that are typically being used in other aerosol compositions, such as butane, pentane, propane and nitrogen generally do not have both of these desirable properties.

Another factor that has to be borne in mind is the safety of aerosol sprays and, in particular, their flammability. One advantage of chlorofluorocarbons as aerosol propellants has been their non-flammability, and one disadvantage of the alternative propellants, such as butane and propane, has been their flammability. One method of measuring flammability of aerosol sprays is the so-called "flame extension" method, in which an aerosol is sprayed sideways into a candle flame at a distance of 150mm from the flame, and the resulting side extension of the flame is measured. According to the industry guidelines issued by the British Aerosol Manufacturers Association (BAMA), the flame extension should not exceed 450mm.

US Patent 4 134 968 discloses an areosol composition comprising a liquid mixture of hydrocarbon propellant, an alcoholic solvent and, to reduce flammability, water and methylene chloride and/or 1,1,1-trichloroethane, wherein the liquid mixture forms a single phase consisting essentially of 5-30% by weight of the hydrocarbon propellant, 5-30% by weight of water, 10-40% by weight methylene chloride and/or 1,1,1,trichloroethane, and 37-80% by weight of ethanol, n-propanol or isopropanol.

Swiss Patent Application 634 480 discloses a pressurised aerosol formulation in the form of a homogeneous solution comprising at least 50% by weight of non-combustible materials and contains as the propellant gases, carbon dioxide and dimethyl ether, and as non-combustible constituents, at least water, carbon dioxide, methylene chloride and/or 1,1,1-trichloroethane.

There has therefore been a desire to produce an aerosol freeze spray which avoids the use of chlorofluorocarbons and satisfies the safety guidelines on flammability.

The present invention now provides an aerosol composition comprising:

(i) an organic substance, other than a chlorofluorocarbon, having a boiling point within the range of from 10°C to 40°C, and

(ii) an organic substance, other than a chlorofluorocarbon, having a boiling point within the range of from -45°C to -10°C, or having a vapour pressure within the range of from $1.034 \times 10^5$ Pa to $7.928 \times 10^5$ Pa (Pascals @ 20°C).

in a ratio of from 2.5 to 6 parts by volume of component (i) per 1 part by volume of component (ii) (or from 3 to 7 parts by weight of component (i) per 1 part by weight of component (ii)), subject to the proviso that the organic substance (i) is not methylene chloride.

Such a composition avoids the use of chlorofluorocarbons and has been found to have a good freeze-cooling effect on the patient's skin while still satisfying the safety guidelines regarding flammability.

Component (i) is an organic substance, other than a chlorofluorocarbon, having a boiling point within the range of from 10°C to 40°C, preferably from 20°C to 35°C, especially from 25°C to 35°C. This component serves primarily as a coolant.

A preferred substance suitable for use as component (i) is isopentane (2-methylbutane; boiling point approx. 28°C). Other suitable substances include, for example, n-pentane (boiling point 36°C) and diethyl ether (ethoxyethane; boiling point 35°C). Two or more such substances may be used together as component (i).

Component (ii) is an organic substance, other than a chlorofluorocarbon, having a boiling point within the range of from -45°C to -10°C, preferably from -30°C to -20°C, especially from -26°C to -20°C, or having a vapour pressure (at 20°C) within the range of from $1.034 \times 10^5$ Pa to $7.928 \times 10^5$ Pa, preferably from $2.068 \times 10^5$ Pa to $4.481 \times 10^5$ Pa, especially from $3.792 \times 10^5$ Pa to $4.481 \times 10^5$ Pa. This component serves primarily as a propellant.

A preferred substance for use as component (ii) is dimethyl ether (methoxymethane; boiling point -25°C).

2

Other suitable substances include, for example, partially hydrogenated chlorofluorocarbons, e.g. HCFC-22, and hydrocarbons, e.g. butane/propane blends. Two or more such substances may be used together as component (ii).

Components (i) and (ii) are used in a ratio (ii):(i) of from 1:2.5 to 1:6 by volume, or from 1:3 to 1:7 by weight; preferably from 1:3 to 1:5.5 by volume, or from 1:3 to 1:5 by weight; for example from 1:3 to 1:4 by volume or 1:3 to 1:4 by weight, or from 1:4 to 1:5 by volume or 1:4 to 1:5 by weight.

Components (i) and (ii) together suitably comprise from 65% to 100% by weight of the total aerosol composition. Components (i) and (ii) together suitably comprise from 65% to 100% by volume of the total aerosol composition.

The aerosol composition according to the invention may additionally comprise a pharmaceutically active substance. Examples of suitable such substances include a topical analgesic agent, for example, salicylic acid, a salicylic acid ester, acetylsalicylic acid (asprin), ibuprofen, indomethacin, or, preferably, glycol salicylate; an anti-inflammatory agent, for example a corticosteroid, e.g. hydrocortisone; a local anaesthetic agent; and an anti-histamine agent.

Such a pharmaceutically active substance may suitably be present in an amount of up to 15% by volume, or up to 20% by weight, based on the total composition.

The pharmaceutically active substance should preferably be dissolved in the mixture of components (i) and (ii) to give a single-phase mixture. It has been found that, when using, say, glycol salicylate as an analgesic agent, the use of dimethyl ether as the propellant has the advantage of aiding solubilisation of the analgesic agent, whereas isopentane and glycol salicylate alone give a two-phase mixture. The use of other propellants, such as butane, does not have this advantage.

The aerosol composition according to the invention may also comprise an organic solvent, in addition to any organic solvent in component (i) or (ii), suitably in an amount of up to 35% by volume, or up to 35% by weight. Examples of suitable organic solvents include isopropanol and ethanol. While it is by no means an essential component, the presence of an organic solvent can also assist in achieving solubilisation of any pharmaceutically active substances present. In the absence of an organic solvent, it may be necessary, in some circumstances, to increase the proportion of either component (i) or (ii), or both, in order to achieve the desired solubilisation.

The aerosol composition according to the invention may furthermore comprise one or more auxiliary components, for example a perfume or fragrance (e.g. menthol), suitably in an amount of up to 5%, preferably up to 2%, by weight or by volume.

The aerosol composition according to the invention is most suitably discharged from an aerosol container at a discharge rate of from 0.2 to 3.5 g/sec, preferably from 0.2 to 1.0 g/sec, such as from 0.2 to 0.5 g/sec or from 0.45 to 0.85 g/sec. The desired discharge rate can be achieved by suitable choice of valve size within the aerosol spray mechanism in a manner known per se.

Changes in the proportions of coolant and propellant within the composition will also affect the discharge rate, in that a higher proportion of propellant will result in an increased vapour pressure within the aerosol container, and may induce a higher discharge rate.

These factors can readily be varied by a person skilled in the art, who can readily choose a combination of coolant:propellant ratio and valve size to give a discharge rate such that flammability safety requirements are satisfied.

Aerosol containers often have both a liquid-phase tap and a vapour-phase tap, that is to say, one outlet extending into the liquid phase within the container (usually a tube extending to the base of the container) and one outlet from the vapour phase at the top of the container. It has, however, been found advantageous for the aerosol composition according to the present invention to be dispensed from a container having only a liquid-phase tap. This has been found to have two advantages: first, it reduces consumption of the propellant and thus enables a smaller proportion of that component to be used; and secondly, it results in a less diffuse, heavier, spray, which in turn gives an improved cooling effect on the skin.

The following Examples 1-7 illustrate the present invention.

The components listed in each example were mixed in the proportions shown and filled into an aerosol container in a conventional manner

Example 1

|  | % w/w |
|---|---|
| isopentane | 60.06 (84.03ml) |
| dimethyl ether | 19.71 (25.64ml) |
| isopropanol | 9.18 (10.21ml) |
| glycol salicylate | 9.89 ( 9.89ml) |
| menthol | 1.15 ( 1.0g ) |

The composition of Example 1 was prepared by mixing together the quantities shown in parenthesis. When discharged from an aerosol container at a discharge rate of about 0.35 g/sec, it gave a flame extension (determined as specified above) not exceeding 450nm.

Example 2

|  | % w/w |
|---|---|
| isopentane | 84.60 |
| dimethyl ether | 15.40 |

Example 3

|  | % w/w |
|---|---|
| isopentane | 60.72 |
| dimethyl ether | 20.06 |
| isopropanol | 9.22 |
| methyl salicylate | 10.00 |

Example 4

|  | % w/w |
|---|---|
| isopentane | 46.62 |
| dimethyl ether | 18.61 |
| ethanol | 33.27 |
| methanol | 0.50 |
| indomethacin | 1.0 |

Example 5

|  | % w/w |
|---|---|
| isopentane | 60.91 |
| dimethyl ether | 27.69 |
| ethanol | 8.32 |
| methanol | 0.50 |
| glycol salicylate | 8.58 |

Example 6

|  | % w/w |
|---|---|
| isopentane | 65.54 |
| dimethyl ether | 16.92 |
| isopropanol | 7.96 |
| glycol salicylate | 8.58 |
| menthol | 1.00 |

Example 7

|  | % w/w |
|---|---|
| isopentane | 67.77 |
| dimethyl ether | 14.41 |
| isopropanol | 6.82 |
| glycol salicylate | 10.00 |
| menthol | 1.00 |

When the compositions of Examples 6 and 7 were discharged from an aerosol container at a discharge rate of about 0.75 g/sec, each gave a flame extension (determined as specified above) not exceeding 450 mm.

**Claims**

**Claims for the following Contracting States: DK, DE, GB, FR, IT, NL, SE, LI, CH, BE, AT, LU, GR**

1. An aerosol composition comprising:
   (i) an organic substance, other than a chlorofluorocarbon, having a boiling point within the range of from 10°C to 40°C, and
   (ii) an organic substance, other than a chlorofluorocarbon, having a boiling point within the range of from -45°C to -10°C, or having a vapour pressure within the range of from $1.034 \times 10^5$ Pa to $7.928 \times 10^5$ Pa (Pascals @ 20°C) (15 to 115 psig, at 20°C);
   in a ratio of from 2.5 to 6 parts by volume of component (i) per 1 part by volume of component (ii) (or from 3 to 7 parts by weight of component (i) per 1 part by weight of component (ii)), subject to the proviso that the organic substance (i), is not methylene chloride.

2. A composition as claimed in claim 1 wherein component (i) comprises isopentane, n-pentane, diethyl ether, or mixtures of any two or more thereof, and component (ii) comprises dimethyl ether, a hydrocarbon

5

or mixtures of any two or more thereof.

3. A composition as claimed in claim 1 or 2 wherein components (i) and (ii) are present in a ratio (ii):(i) of from 1:3 to 1:5.5 by volume or from 1:3 to 1:4 by weight.

4. A composition as claimed in claim 3 wherein the ratio (ii):(i) is from 1:4 to 1:5 by volume or 1:4 to 1:5 by weight.

5. A composition as claimed in any preceding claim wherein components (i) and (ii) together comprise from 65 to 100% by weight or from 65 to 100% by volume of the total aerosol composition.

6. A composition as claimed in any preceding claim further comprising a pharmaceutically active substance.

7. A composition as claimed in claim 6 wherein the pharmaceutically active substance is a topical analgesic agent, an anti-inflammatory agent, a local anaesthetic agent or an anti-histamine agent.

8. A composition as claimed in claim 7 wherein the pharmaceutically active substance is salicylic acid or an ester thereof, acetyl salicylic acid, glycol salicylate, ibuprofen, indomethacin, or hydrocortisone.

9. A composition as claimed in any one of claims 6 to 8 wherein the pharmaceutically active substance comprises up to 15% by volume or up to 20% by weight of the total composition.

10. A composition as claimed in any preceding claim further comprising an organic solvent in addition to any organic solvent in component (i) or (ii).

11. A composition as claimed in claim 10 comprising up to 35% by volume or up to 35% by weight of an organic solvent in addition to any organic solvent in component (i) or (ii).

12. A composition as claimed in claim 10 or 11 containing isopropanol or ethanol as the said organic solvent.

13. An aerosol container containing a composition as claimed in any preceding claim having a discharge rate of from 0.2 to 3.5 g/s.

14. An aerosol container as defined in claim 13 having only a liquid-phase tap.

15. An aerosol composition comprising:
(i) an organic substance, other than a chlorofluorocarbon, having a boiling point within the range of from 10°C to 40°C, and
(ii) an organic substance, other than a chlorofluorocarbon, having a boiling point within the range of from -45°C to -10°C, or having a vapour pressure within the range of from 1.034 x $10^5$ Pa to 7.928 x $10^5$ Pa (Pascals @ 20°C) (15 to 115 psig, at 20°C)
in a ratio of from 2.5 to 6 parts by volume of component (i) per 1 part by volume of component (ii) (or from 3 to 7 parts by weight of component (i) per 1 part by weight of component (ii), for use in therapy, with the proviso that the organic substance (i) is not methylene chloride.

16. The use of an aerosol composition comprising:
(i) an organic substance, other than a chlorofluorocarbon, having a boiling point within the range of from 10°C to 40°C, and
(ii) an organic substance, other than a chlorofluorocarbon, having a boiling point within the range of from -45°C to -10°C, or having a vapour pressure within the range of from 1.034 x $10^5$ Pa to 7.928 x $10^5$ Pa (Pascals @ 20°C) (15 to 115 psig, at 20°C. in a ratio of from 2.5 to 6 parts by volume of component (i) per 1 part by volume of component (ii) (or from 3 to 7 parts by weight of component (i) per 1 part by weight of component (ii), for the manufacture of a medicament for application as a freeze spray.

**Claims for the following Contracting State: ES**

1. A process for the preparation of an aerosol composition comprising:
(i) an organic substance, other than a chlorofluorocarbon, having a boiling point within the range of from 10°C to 40°C, and
(ii) an organic substance, other than a chlorofluorocarbon, having a boiling point within the range of from -45°C to -10°C, or having a vapour pressure within the range of from 1.034 x $10^5$ Pa to 7.928 x $10^5$ Pa (Pascals @ 20°C) (15 to 115 psig, at 20°C);

in a ratio of from 2.5 to 6 parts by volume of component (i) per 1 part by volume of component (ii) (or from 3 to 7 parts by weight of component (i) per 1 part by weight of component (ii)), which process comprises the admixture of components (i) and (ii), subject to the proviso that the organic substance (i), is not methylene chloride.

2. A process as claimed in claim 1 wherein component (i) comprises isopentane, n-pentane, diethyl ether or methylene chloride, or mixtures of any two or more thereof.

3. A process as claimed in claim 1 or 2 wherein component (ii) comprises dimethyl ether, a hydrocarbon or mixtures of any two or more thereof.

4. A process as claimed in claim 1, 2 or 3 wherein components (i) and (ii) are mixed together in a ratio (ii):(i) of from 1:3 to 1:5.5 by volume or from 1:3 to 1:4 by weight.

5. A process as claimed in claim 4 wherein the ratio (ii):(i) is from 1:4 to 1:5 by volume or 1:4 to 1:5 by weight.

6. A process as claimed in any preceding claim wherein components (i) and (ii) together comprise from 65 to 100% by weight or from 65 to 100% by volume of the total aerosol composition.

7. A process as claimed in any preceding claim further comprising the admixture of a pharmaceutically active substance with components (i) and (ii).

8. A process as claimed in claim 7 wherein the pharmaceutically active substance is a topical analgesic agent, an anti-inflammatory agent, a local anaesthetic agent or an anti-histamine agent.

9. A process as claimed in claim 8 wherein the pharmaceutically active substance is salicylic acid or an ester thereof, acetyl salicylic acid, glycol salicylate, ibuprofen, indomethacin, or hydrocortisone.

10. A process as claimed in any one of claims 7 to 9 wherein the pharmaceutically active substance comprises up to 15% by volume or up to 20% by weight of the total composition.

11. A process as claimed in any preceding claim further comprising the admixture of an organic solvent in addition to any organic solvent in component (i) or (ii).

12. A process as claimed in claim 11 wherein the said organic solvent comprises up to 35% by volume or up to 35% by weight of the total composition.

13. A process as claimed in claim 11 or 12 wherein the said organic solvent comprises isopropanol or ethanol.

14. A process as claimed in any preceding claim further comprising the admixture of a perfume.

15. A process as claimed in claim 14 wherein the perfume comprises up to 5% by weight or volume of the total composition.

16. A process as claimed in claim 14 or 15 wherein the perfume is menthol.

17. An aerosol container containing a composition as claimed in any preceding claim having a discharge rate of from 0.2 to 3.5 g/s.

18. An aerosol container as defined in claim 17 having only a liquid-phase tap.

19. An aerosol composition comprising:
(i) an organic substance, other than a chlorofluorocarbon, having a boiling point within the range of from 10°C to 40°C, and
(ii) an organic substance, other than a chlorofluorocarbon, having a boiling point within the range of from -45°C to -10°C, or having a vapour pressure within the range of from 1.034 x $10^5$ Pa to 7.928 x $10^5$ Pa (Pascals @ 20°C (15 to 115 psig, at 20°C);
in a ratio of from 2.5 to 6 parts by volume of component (i) per 1 part by volume of component (ii) (or from 3 to 7 parts by weight of component (i) per 1 part by weight of component (ii), for use in therapy, subject to the proviso that the organic substance (i), is not methylene chloride.

**Patentansprüche**

**Patentansprüche für folgende Vertragsstaaten : DK, DE, GB, FR, IT, NL, SE, LI, CH, BE, AT, LU, GR**

1. Aerosol-Zusammensetzung, umfassend:
   (i) eine von Fluorchlorkohlenwasserstoff verschiedene organische Substanz mit einem Siedepunkt im Bereich von 10°C bis 40°C, und
   (ii) eine von Fluorchlorkohlenwasserstoff verschiedene organische Substanz mit einem Siedepunkt im Bereich von -45°C bis -10°C, oder mit einem Dampfdruck im Bereich von $1.034 \times 10^5$ Pa bis $7.928 \times 10^5$ Pa (Pascal bei 20°C; 15 bis 115 psig, bei 20°C),
   in einem Volumenverhältnis der Komponenten (i):(ii) von 2.5:1 bis 6:1 (oder in einem Gewichtsverhältnis der Komponenten (i):(ii) von 3:1 bis 7:1), mit der Maßgabe, daß die organische Substanz (i) nicht Methylenchlorid ist.

2. Zusammensetzung nach Anspruch 1, wobei die Komponente (i) Isopentan, n-Pentan, Diethylether oder Gemische von zwei oder mehreren davon und die Komponente (ii) Dimethylether, ein Kohlenwasserstoff oder deren Gemische umfaßt.

3. Zusammensetzung nach Anspruch 1 oder 2, wobei die Komponenten (ii) und (i) in einem Volumenverhältnis (ii):(i) von 1:3 bis 1:5.5 oder in einem Gewichtsverhältnis von 1:3 bis 1:4 vorliegen.

4. Zusammensetzung nach Anspruch 3, wobei das Volumenverhältnis und das Gewichtsverhältnis (ii):(i) 1:4 bis 1:5 ist.

5. Zusammensetzung nach einem der vorstehenden Ansprüche, wobei die Komponenten (i) und (ii) zusammen 65 bis 100 Gew.-% oder 65 bis 100 Volumen-% der gesamten Aerosolzusammensetzung umfassen.

6. Zusammensetzung nach einem der vorstehenden Ansprüche, die zusätzlich eine pharmazeutisch aktive Substanz umfaßt.

7. Zusammensetzung nach Anspruch 6, wobei die pharmazeutisch aktive Substanz ein Lokalanalgetikum, ein Antiphlogistikum, ein Lokalanaesthetikum oder ein Antihistaminikum ist.

8. Zusammensetzung nach Anspruch 7, wobei die pharmazeutisch aktive Substanz Salicylsäure oder ein Ester davon, Acetylsalicylsäure, Glykolsalicylat, Ibuprofen, Indomethacin oder Hydrocortison ist.

9. Zusammensetzung nach einem der Ansprüche 6 bis 8, wobei die pharmazeutisch aktive Substanz bis zu 15 Volumen-% oder bis zu 20 Gew.-% der Gesamtzusammensetzung umfaßt.

10. Zusammensetzung nach einem der vorstehenden Ansprüche, die zusätzlich zu einem organischen Lösungsmittel in Komponente (i) oder (ii) ein organisches Lösungsmittel enthält.

11. Zusammensetzung nach Anspruch 10, die zusätzlich zu einem organischen Lösungsmittel in Komponente (i) oder (ii) bis zu 35 Volumen-% oder bis zu 35 Gew.-% eines organischen Lösungsmittels enthält.

12. Zusammensetzung nach Anspruch 10 oder 11, die als zusätzliches organisches Lösungsmittel Isopropanol oder Ethanol enthält.

13. Aerosolbehälter, der eine Zusammensetzung nach einem der vorstehenden Ansprüche mit einer Abgaberate von 0.2 bis 3.5 g/s enthält.

14. Aerosolbehälter nach Anspruch 13, der nur einen Auslaß besitzt, der sich in die flüssige Phase erstreckt (a liquid-phase tap).

15. Aerosolzusammensetzung, umfassend:
    (i) eine von Fluorchlorkohlenwasserstoff verschiedene organische Substanz mit einem Siedepunkt im Bereich von 10°C bis 40°C, und

(ii) eine von Fluorchlorkohlenwasserstoff verschiedene organische Substanz mit einem Siedepunkt im Bereich von -45°C bis -10°C oder mit einem Dampfdruck im Bereich von $1.034 \times 10^5$ Pa bis $7.928 \times 10^5$ Pa (Pascal bei 20°C; 15 bis 115 psig, bei 20°C),

in einem Volumenverhältnis der Komponenten (i):(ii) von 2.5:1 bis 6:1 (oder in einem Gewichtsverhältnis der Komponenten (i):(ii) von 3:1 bis 7:1), zu therapeutischen Zwecken, mit der Maßgabe, daß die organische Substanz (i) nicht Methylenchlorid ist.

16. Verwendung einer Aerosolzusammensetzung, umfassend:

(i) eine von Fluorchlorkohlenwasserstoff verschiedene organische Substanz mit einem Siedepunkt im Bereich von 10°C bis 40°C, und

(ii) eine von Fluorchlorkohlenwasserstoff verschiedene organische Substanz mit einem Siedepunkt im Bereich von -45°C bis - 10°C oder mit einem Dampfdruck im Bereich von $1.034 \times 10^5$ Pa bis $7.928 \times 10^5$ Pa (Pascal bei 20°C; 15 bis 115 psig, bei 20°C),

in einem Volumenverhältnis der Komponenten (i):(ii) von 2.5:1 bis 6:1 (oder in einem Gewichtsverhältnis der Komponenten (i):(ii) von 3:1 bis 7:1), zur Herstellung eines Arzneimittels zum Anwenden als Vereisungsspray.

**Patentansprüche für folgenden Vertragsstaat : Es**

1. Verfahren zur Herstellung einer Aerosolzusammensetzung, umfassend:

(i) eine von Fluorchlorkohlenwasserstoff verschiedene organische Substanz mit einem Siedepunkt im Bereich von 10°C bis 40°C, und

(ii) eine von Fluorchlorkohlenwasserstoff verschiedene organische Substanz mit einem Siedepunkt im Bereich von -45°C bis -10°C, oder mit einem Dampfdruck im Bereich von $1.034 \times 10^5$ Pa bis $7.928 \times 10^5$ Pa (Pascal bei 20°C; 15 bis 115 psig, bei 20°C),

in einem Volumenverhältnis der Komponenten (i):(ii) von 2.5:1 bis 6:1 (oder in einem Gewichtsverhältnis der Komponenten (i):(ii) von 3:1 bis 7:1), wobei das Verfahren das Vermischen der Komponenten (i) und (ii) umfaßt, mit der Maßgabe, daß die organische Substanz (i) nicht Methylenchlorid ist.

2. Verfahren nach Anspruch 1, wobei die Komponente (i) Isopentan, n-Pentan, Diethylether oder Gemische von zwei oder mehreren davon umfaßt.

3. Verfahren nach Anspruch 1 oder 2, wobei die Komponente (ii) Dimethylether, ein Kohlenwasserstoff oder deren Gemische umfaßt.

4. Verfahren nach Anspruch 1, 2 oder 3, wobei die Komponenten (ii) und (i) zusammen in einem Volumenverhältnis (ii):(i) von 1:3 bis 1:5.5 oder in einem Gewichtsverhältnis von 1:3 bis 1:4 gemischt werden.

5. Verfahren nach Anspruch 4, wobei das Volumenverhältnis und das Gewichtsverhältnis (ii):(i) 1:4 bis 1:5 ist.

6. Verfahren nach einem der vorstehenden Ansprüche, in dem die Komponenten (i) und (ii) zusammen 65 bis 100 Gew.-% oder 65 bis 100 Volumen-% der gesamten Aerosolzusammensetzung umfassen.

7. Verfahren nach einem der vorstehenden Ansprüche, dadurch gekennzeichnet, daß zusätzlich eine pharmazeutisch aktive Substanz mit den Komponenten (i) und (ii) vermischt ist.

8. Verfahren nach Anspruch 7, wobei die pharmazeutisch aktive Substanz ein Lokalanalgetikum, ein Antiphlogistikum, ein Lokalanaesthetikum oder ein Antihistaminikum ist.

9. Verfahren nach Anspruch 8, wobei die pharmazeutisch aktive Substanz Salicylsäure oder ein Ester davon, Acetylsalicylsäure, Glykolsalicylat, Ibuprofen, Indomethacin oder Hydrocortison ist.

10. Verfahren nach einem der Ansprüche 7 bis 9, wobei die pharmazeutisch aktive Substanz bis zu 15 Volumen-% oder bis zu 20 Gew.-% der Gesamtzusammensetzung umfaßt.

11. Verfahren nach einem der vorstehenden Ansprüche, das zusätzlich das Vermischen eines organischen Lösungsmittels mit einem der organischen Lösungsmittel in der Komponente (i) oder (ii) umfaßt.

12. Verfahren nach Anspruch 11, in dem das zusätzliche organische Losungsmittel bis zu 35 Volumen-% oder bis zu 35 Gew.-% der Gesamtzusammensetzung umfaßt.

13. Verfahren nach Anspruch 11 oder 12, wobei das zusätzliche organische Lösungsmittel Isopropanol oder Ethanol umfaßt.

14. Verfahren nach einem der vorstehenden Ansprüche, das zusätzlich das Zumischen eines Duftstoffs umfaßt.

15. Verfahren nach Anspruch 14, wobei der Duftstoff bis zu 5 Gew.- oder Volumen-% der Gesamtzusammensetzung umfaßt.

16. Verfahren nach Anspruch 14 oder 15, wobei der Duftstoff Menthol ist.

17. Aerosolbehälter, der eine Zusammensetzung nach einem der vorstehenden Ansprüche mit einer Abgaberate von 0.2 bis 3.5 g/s enthält.

18. Aerosolbehälter nach Anspruch 17, der nur einen Auslaß besitzt, der sich in die flüssige Phase erstreckt (a liquid-phase tap).

19. Aerosolzusammensetzung, umfassend:
(i) eine von Fluorchlorkohlenwasserstoff verschiedene organische Substanz mit einem Siedepunkt im Bereich von 10°C bis 40°C, und
(ii) eine von Fluorchlorkohlenwasserstoff verschiedene organische Substanz mit einem Siedepunkt im Bereich von -45°C bis -10°C oder mit einem Dampfdruck im Bereich von $1.034 \times 10^5$ Pa bis $7.928 \times 10^5$ Pa (Pascal bei 20°C; 15 bis 115 psig, bei 20°C),
in einem Volumenverhältnis der Komponenten (i):(ii) von 2.5:1 bis 6:1 (oder in einem Gewichtsverhältnis der Komponenten (i):(ii) von 3:1 bis 7:1), zu therapeutischen Zwecken, mit der Maßgabe, daß die organische Substanz (i) nicht Methylenchlorid ist.

## Revendications

**Revendications pour les Etats contractants suivants : DK, DE, GB, FR, IT, NL, SE, LI, CH, BE, AT, LU, GR**

1. Composition d'aérosol, comprenant :
(i) une substance organique, autre qu'un chlorofluorocarbure, ayant un point d'ébullition compris dans la gamme de 10°C à 40°C, et
(ii) une substance organique, autre qu'un chlorofluorocarbure, ayant un point d'ébullition compris dans la gamme de -45°C à -10°C, ou une pression de vapeur comprise dans la gamme de $1,034 \times 10^5$ Pa à $7,928 \times 10^5$ Pa (Pascals à 20°C) (15 à 115 psig, à 20°C);
selon un rapport de 2,5 à 6 parties en volume du composant (i) par partie en volume de composant (ii) (ou de 3 à 7 parties en poids de composant (i) par partie en poids de composant (ii)), à condition que la substance organique (i) ne soit pas le chlorure de méthylène.

2. Compositon suivant la revendication 1, dans laquelle le composant (i) comprend l'isopentane, le n-pentane, l'éther diéthylique ou des mélanges de deux ou plusieurs de ceux-ci et le composant (ii) comprend l'éther diméthylique, un hydrocarbure ou des mélanges de deux ou plusieurs de ceux-ci.

3. Composition suivant les revendications 1 ou 2, dans laquelle les composants (i) et (ii) sont présents selon un rapport (ii) : (i) de 1:8 à 1:5,5 en volume, ou de 1:8 à 1:4 en poids.

4. Compositon suivant le revendication 3, dans laquelle le rapport (ii) : (i) est de 1:4 à 1:5 en volume, ou de 1:4 à 1:5 en poids.

5. Composition suivant l'une quelconque des revendications précédentes, dans laquelle les composants (i) et (ii) constituent ensemble de 65 à 100% en poids ou de 65 à 100% en volume de la composition totale d'aérosol.

**6.** Composition suivant l'une quelconque des revendications précédentes, comprenant de plus une substance active du point de vue pharmaceutique.

**7.** Composition suivant la revendication 6, dans laquelle la substance active du point de vue pharmaceutique est un agent analgésique topique, un agent anti-inflammatoire, un agent anésthésique local ou un agent histaminique.

**8.** Composition suivant la revendication 7, dans laquelle la substance active du point de vue pharmaceutique est l'acide salicylique ou un ester de celui-ci, l'acide acétyl salicylique, le salicylate de glycol, l'ibuprofène, l'indométhacine ou l'hydrocortisone.

**9.** Compositon suivant l'une quelconque des revendications 6 à 8, dans laquelle la substance active du point de vue pharmaceutique représente jusqu'à 15% en volume ou jusqu'à 20% en poids de la composition totale.

**10.** Composition suivant l'une quelconque des revendications précédentes, comprenant de plus un solvant organique ou plus d'un quelconque solvant organique dans les composants (i) ou (ii).

**11.** Composition suivant la revendication 10, dans laquelle ce solvant organique représente jusqu'à 35 % en volume ou jusqu'à 35% en poids, en plus d'un quelconque solvant organique dans les composants (i) ou (ii).

**12.** Composition suivant les revendications 10 ou 11, dans laquelle ce solvant organique comprend l'isopropanol ou l'éthanol.

**13.** Récipient pour aérosol contenant une composition suivant l'une quelconque des revendications précédentes, ayant un débit de 0,2 à 3,5 g/s.

**14.** Récipient pour aérosol suivant le revendication 13, n'ayant qu'une prise d'alimentation dans la phase liquide.

**15.** Composition d'aérosol, comprenant:
(i) une substance organique, autre qu'un chlorofluorocarbure, ayant un point d'ébullition compris dans la gamme de 10°C à 40°C, et
(ii) une substance organique, autre qu'un chlorofluorocarbure, ayant un point d'ébullition compris dans la gamme de -45°C à -10°C, ou une pression de vapeur comprise dans la gamme de 1,034 x 10$^5$ Pa à 7,928 x 10$^5$ Pa (Pascals à 20°C) (15 à 115 psig, à 20°C);
selon un rapport de 2,5 à 6 parties en volume du composant (i) par partie en volume de composant (ii) (ou de 3 à 7 parties en poids de composant (i) par parties en poids de composant (ii)), utile en thérapie à condition que la substance organique (i) ne soit pas le chlorure de méthylène.

**16.** Utilisation d'une composition d'aérosol, comprenant :
(i) une substance organique, autre qu'un chlorofluorocarbure, ayant un point d'ébullition compris dans la gamme de 10°C à 40°C, et
(ii) une substance organique, autre qu'un chlorofluorocarbure, ayant un point d'ébullition compris dans la gamme de -45°C à -10°C ou une pression de vapeur comprise dans la gamme de 1,034 ×10$^5$ Pa à 7,928 ×10$^5$ Pa (Pascals à 20°C) (15 à 115 psig, à 20°C);
selon un rapport de 2,5 à 6 parties en volume du composant (i) par partie en volume de composant (ii) (ou de 8 à 7 parties en poids de composant (i) par partie en poids de composant (ii)), pour la fabrication d'un médicament à appliquer sous forme de spray glaçant.

**Revendications pour l'Etat contractant suivant : ES**

**1.** Procédé pour la préparation d'une composition d'aérosol comprenant :
(i) une substance organique, autre qu'un chlorofluorocarbure, ayant un point d'ébullition compris dans la gamme de 10°C à 40°C, et
(ii) une substance organique, autre qu'un chlorofluorocarbure, ayant un point d'ébullition compris dans la gamme de -45°C à -10°C, ou une pression de vapeur comprise dans la gamme de 1,034 x 10$^5$ Pa à 7,928 x 10$^5$ Pa (Pascals à 20°C) (15 à 115 psig, à 20°C);

selon un rapport de 2,5 à 6 parties en volume du composant (i) par partie en volume de composant (ii) (ou de 3 à 7 parties en poids de composant (i) par partie en poids de composant (ii), lequel procédé comprend le mélange des composants (i) et (ii), à condition que la substance organique (i) ne soit pas le chlorure de méthylène.

2. Procédé suivant la revendication 1, dans lequel le composant (i) comprend l'isopentane, le n-pentane, l'éther diéthylique ou des mélanges de deux ou plusieurs de ceux-ci.

3. Procédé suivant les revendications 1 ou 2, dans lequel le composant (ii) comprend l'éther diéthylique, un hydrocarbure ou des mélanges de deux ou plusieurs de ceux-ci.

4. Procédé suivant les revendications 1, 2 ou 3, dans lequel les composants (i) et (ii) sont mélangés selon un rapport (ii) : (i) de 1:3 à 1:5,5 en volume, ou de 1:3 à 1:4 en poids.

5. Procédé suivant la revendication 4, dans lequel le rapport (ii) : (i) est de 1:4 à 1:5 en volume, ou de 1:4 à 1:5 en poids.

6. Procédé suivant l'une quelconque des revendications précédentes, dans lequel les composants (i) et (ii) constituent ensemble de 65 à 100% en poids ou de 95 à 100% en volume de la composition totale d'aérosol.

7. Procédé suivant l'une quelconque des revendications précédentes, comprenant de plus le mélange d'une substance active du point de vue pharmaceutique avec les composants (i) et (ii).

8. Procédé suivant la revendication 7, dans lequel la substance active du point de vue pharmaceutique est un agent analgésique topique, un agent anti-inflammatoire, un agent anesthésique local ou un agent-histaminique.

9. Procédé suivant la revendication 8, dans lequel la substance active du point de vue pharmaceutique est l'acide salicylique ou un ester de celui-ci, l'acide acétyl salicylique, le salicylate de glycol, l'ibuprofène, l'indométhacine ou l'hydrocortisone.

10. Procédé suivant l'une quelconques des revendications 7 à 9, dans lequel la substance active du point de vue pharmaceutique représente jusqu'à 15% en volume ou jusqu'à 20% en poids de la composition totale.

11. Procédé suivant l'une quelconque des revendications précédentes, comprenant de plus le mélange d'un solvant organique en plus d'un quelconque solvant organique dans les composants (i) ou (ii).

12. Procédé suivant la revendication 11, dans lequel ce solvant organique représente jusqu'à 35% en volume ou jusqu'à 35% en poids de la composition totale.

13. Procédé suivant les revendications 11 ou 12, dans lequel ce solvant organique comprend l'isopropanol ou l'éthanol.

14. Procédé suivant l'une quelconque des revendications précédentes, comprenant de plus le mélange d'un parfum.

15. Procédé suivant la revendication 14, dans lequel ce parfum représente jusqu'à 5% en poids ou volume de la composition totale.

16. Procédé suivant les revendications 14 ou 15, dans lequel le parfum est le menthol.

17. Récipient pour aérosol contenant une composition suivant l'une quelconque des revendications précédentes, ayant un débit de 0,2 à 3,5 g/s.

18. Récipient pour aérosol suivant la revendication 17, n'ayant qu'une prise d'alimentation dans la phase liquide.

19. Composition d'aérosol, comprenant :
      (i) une substance organique, autre qu'un chlorofluorocarbure, ayant un point d'ébullition compris dans la gamme de 10°C à 40°C, et

EP 0 404 334 B1

(ii) une substance organique, autre qu'un chlorofluorocarbure, ayant un point d'ébullition compris dans la gamme de -45°C à -10°C, ou une pression de vapeur comprise dans la gamme de 1,034 x 10$^5$ Pa à 7,928 x 10$^5$ Pa (Pascals à 20°C) (15 à 115 psig, à 20°C);

selon un rapport de 2,5 à 6 parties en volume du composant (i) par partie en volume de composant (ii) (ou de 3 à 7 parties en poids de composant (i) par partie en poids de composant (ii), utile en thérapie, à condition que la substance organique (i) ne soit pas le chlorure de méthylène.